# EUROPEAN PATENT APPLICATION

(11) **EP 1 731 942 A1**
(43) Date of publication of application: **13.12.2006**
(21) Application number: 06011840.3
(22) Date of filing: 08.06.2006
(51) Int. Cl.: G02B 27/00, G02B 21/00, A61B 19/08, A61B 19/02, A61B 1/00

(54) **Objective protector and microscope observation method**

(30) Priority: 10.06.2005 JP 2005171209
(71) Applicant: Olympus Corporation, Tokyo 151-0072 (JP)
(72) Inventor: Hirata, Tadashi, Hachioji-shi, Tokyo 193-0842 (JP); Fukuyama, Hiroya, Machida-shi, Tokyo 194-0211 (JP); Yamashita, Hideto, Kamiina-gun, Nagano 399-4511 (JP)
(74) Representative: von Hellfeld, Axel

(57) **Abstract**

The present invention reduces or removes the need to clean an objective lens, thus simplifying the observation procedure, prevents bacteria or contamination from adhering to the objective lens, and protects the objective lens from external force that would be exerted during cleaning. The invention includes an optically transparent covering configured to cover at least an end surface of an objective lens; and an attaching mechanism configured to attach the covering to the objective lens.

## Description

### BACKGROUND OF THE INVENTION

### 1. FIELD OF THE INVENTION

The present invention is related to an objective protector and to a microscope observation method.

This application is based on Japanese Patent Application No. 2005-171209, the content of which is incorporated herein by reference.

### 2. DESCRIPTION OF RELATED ART

Various configurations of objective lenses for microscopes have been proposed in the related art (for example, see Japanese Unexamined Patent Application Publication No. 2005-31425).

These objective lenses in the related art are configured such that a plurality of lenses are arranged inside a substantially cylindrical lens barrel. By constructing the lens barrel with a comparatively large diameter and a thick wall, it has high stiffness and can firmly hold a plurality of lenses. Because only a cover glass at the tip is disposed close to the exterior of the sample to be observed, it is possible to keep the outer diameter thereof quite large.

However, in the case of microscope apparatuses for carrying out in-vivo examination of living organisms, it is necessary to insert the tip of the objective lens inside the living organism to carry out observation. In other words, a great burden is placed on the living organism if the degree of invasiveness is high. Therefore, from the viewpoint of observing living organisms with minimal invasiveness, it is necessary to design objective lenses having small-diameter tips.

On the other hand, it is also necessary, in the case of microscope apparatuses for in-vivo examination of living organism such as those described above, to place the tip of the objective lens in contact with the living organism to carry out observation. Therefore, in order to prevent contamination, such as bacteria, or dust from adhering to the tip of the objective lens, it is absolutely essential to clean, disinfect, or sterilize it after use.

However, performing such cleaning, disinfecting, or sterilizing after each use is cumbersome. In addition, if the tip of the objective lens has a small-diameter, there is a risk of deformation of the small-diameter tip during cleaning or the like due to the external force applied, particularly because the rigidity against external forces from the lens barrel is reduced.

### BRIEF SUMMARY OF THE INVENTION

The present invention has been conceived in light of the circumstances described above, and an object thereof is to provide an objective protector and a microscope observation method which can reduce or remove the need to clean an objective lens, thus simplifying the observation procedure, which can prevent bacteria or contamination from adhering to the objective lens, and which can protect the objective lens from external force that would be exerted during cleaning.

In order to realize the object described above, the present invention provides the following solutions.

A first aspect of the present invention is an objective protector comprising an optically transparent covering configured to cover at least an end surface of an objective lens; and an attaching mechanism configured to attach the covering to the objective lens.

According to the above-described aspect, the covering is attached to at least the end surface of the objective lens by the action of the attaching mechanism. Because the covering is formed of an optically transparent material, light enters and emerges via the end surface of the objective lens without being disrupted. In addition, because at least the end surface of the objective lens is prevented from directly making contact with the specimen, such as a living organism, by the covering, it is possible to prevent the objective lens from being contaminated by bacteria, dirt, or the like. Also, merely by removing the covering after observation, it is possible to eliminate the need for disinfecting the objective lens.

In the aspect of the invention described above, the covering may include a cover member that is brought into close contact with the end surface of the objective lens.

By doing so, the end surface of the objective lens can be protected from bacteria, contamination, or the like by the cover member, and it is possible to allow light from the specimen to enter the objective lens without any losses.

In the aspect of the invention described above, the covering may include a membrane member that is brought into close contact with the end surface of the objective lens and that is made from a plastic material selected from the group consisting of polyethylene, polyvinylidene chloride, polystyrene, polypropylene, and polycarbonate.

With this configuration, it is possible to deform the membrane member so that it follows the shape of the end surface of the objective lens, thus making it easier to bring into close contact therewith. Therefore, it is possible to efficiently prevent bacteria and contamination from adhering to at least the end surface of the objective lens, without changing the optical characteristics at the tip of the objective lens. In addition, by using a relative large membrane, it is possible to easily protect a large portion of the objective lens, not just the end surface thereof.

In the aspect of the invention described above, a circumferential groove may be formed in an outer circumferential surface of the objective lens, and the attaching mechanism may include a constricting member made from a ring-shaped elastic body configured to engage with the circumferential groove to pinch the membrane member between the constricting member and the outer circumferential surface of the objective lens.

With this configuration, the membrane member is disposed as to cover the outer circumferential surface of the objective lens, and the constricting member is engaged with the circumferential groove from the outer side thereof. Doing so allows the membrane member of the objective lens to be easily secured to the outer circumferential surface of the objective lens by means of the elastic force of the constricting member.

In the aspect of the invention described above, the attaching mechanism may include a ring-shaped member secured to the outer circumferential surface of the objective lens; and a ring-shaped constricting member configured to engage with the ring-shaped member to pinch the membrane member between the constricting member and the ring-shaped member.

Therefore, the membrane member can be easily secured to the outer circumferential surface of the objective lens even if the objective lens has a small diameter or if it is difficult to form a circumferential groove in the outer circumferential surface thereof due to its thin structure.

The aspect of the invention described above may further comprise a tension-applying mechanism configured to apply tension to the membrane member so that the membrane member is brought into close contact with the end surface of the objective lens.

With this configuration, it is possible to bring the membrane member into close contact with the end surface of the objective lens simply by applying tension to the membrane member by the action of the tension-applying mechanism. Therefore, it is possible to effectively protect the end surface of the objective lens without changing the optical characteristics at the tip of the objective lens.

A second aspect of the present invention is an objective protector comprising an optically transparent covering configured to cover at least an end surface of a fiber bundle, which is disposed at the tip of an objective lens; and an attaching mechanism configured to attach the covering to the fiber bundle.

According to the above-described aspect, the covering is attached to at least the end surface of the fiber bundle by the attaching mechanism. Because the covering member is formed of an optically transparent material, light can enter and exit via the end surface of the fiber bundle without being disturbed. In addition, because at least the end surface of the fiber bundle is prevented from coming into direct contact with the specimen, such as a living organism, by the covering, it is possible to prevent the tip of the fiber bundle from becoming contaminated by bacteria, dirt, or the like. Moreover, simply by removing the covering after observation, it is possible to eliminate the need to disinfect the fiber bundle.

A third aspect of the present invention is a microscope observation method comprising covering an objective lens provided in a microscope with a membrane member made of an optically transparent plastic material selected from the group consisting of polyethylene, polyvinylidene chloride, polystyrene, polypropylene, and polycarbonate; and carrying out observation with the membrane member being in close contact with an end surface of the objective lens.

According to the above-described aspect, because the objective lens is covered with the membrane member, it is possible to prevent bacteria or contamination from adhering to the objective lens, even when the objective lens is brought into contact with the specimen or inserted inside the specimen. Therefore, it is possible to eliminate the need to clean the objective lens after observation. In such a case, by bringing the membrane member into close contact with the end surface of the objective lens, it is possible to carry out observation with no effect on the optical characteristics.

The present invention provides an advantage in that it can reduce or remove the need to clean the objective lens, prevent bacteria or contamination from adhering to the objective lens, and protect the objective lens from external force which would be exerted thereon during cleaning.

### BRIEF DESCRIPTION OF THE SEVERAL VIEWS OF THE DRAWINGS

Fig. 1 is a plan view showing an objective protector according to a first embodiment of the present invention.
Fig. 2 is a partially magnified view showing the relationship between a small-diameter tip and a covering, when the objective protector in Fig. 1 is attached.
Fig. 3 is a plan view showing an objective protector according to a second embodiment of the present invention.
Fig. 4 is a longitudinal sectional view showing an objective protector according to a third embodiment of the present invention.
Fig. 5 is a longitudinal sectional view showing a modification of the objective protector in Fig. 4.
Fig. 6 is a longitudinal sectional view showing a modification of the covering.
Fig. 7 is a plan view showing a modification of the objective protector when attached only to a small-diameter tip.
Fig. 8 is a schematic diagram showing an objective protector according to a fourth embodiment of the present invention.
Fig. 9 is a perspective view showing an attaching mechanism of the objective protector in Fig. 8.
Fig. 10 is a longitudinal sectional view showing a modification of a structure for attaching a fiber bundle.

### DETAILED DESCRIPTION OF THE INVENTION

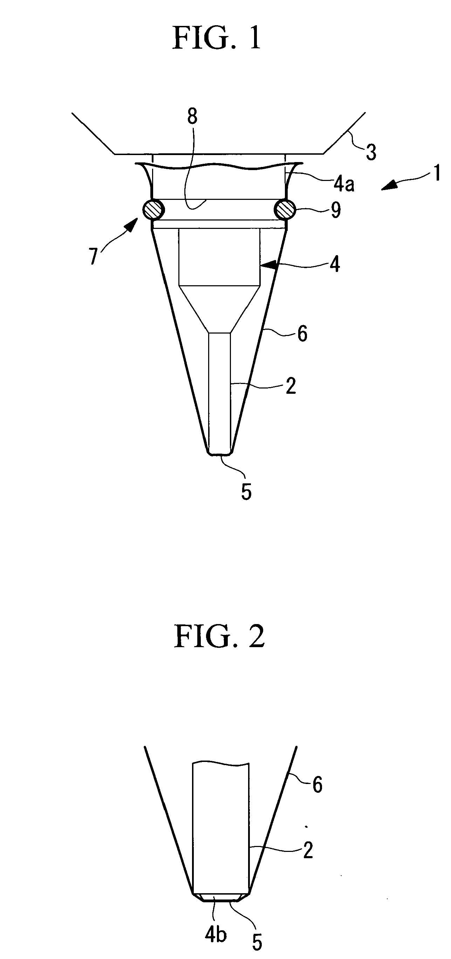

An objective protector 1 according to a first embodiment of present invention will be described below with reference to Figs. 1 and 2.

As shown in Fig. 1, the objective protector 1 of this embodiment protects an objective lens 4 which has a small-diameter tip 2 at the front end thereof and the rear end of which is attached to a microscope main body 3.

This objective protector 1 includes an optically transparent covering 6, which covers at least an end surface 5 of the objective lens 4, and an attaching mechanism 7 for attaching the covering 6 to the objective lens 4.

The covering 6 is formed of a membrane member made from an optically transparent plastic material, for example, polyethylene, polyvinylidene chloride, polystyrene, polypropylene, or polycarbonate. The thickness of the covering 6 is, for example, from several micrometers to several hundred micrometers.

The attaching mechanism 7 is formed, for example, of a circumferential groove 8, which is formed around the entire circumference of the outer circumferential surface of a large-diameter portion 4a of the objective lens 4, and an O-ring constricting member 9 which can engage with the circumferential groove 8.

A microscope observation method using the objective protector 1 according to this embodiment, having such a configuration, will be described below.

As shown in Fig. 1, to attach the objective protector 1 according to this embodiment to the objective lens 4, first the part from the small-diameter tip 2 of the objective lens 4 to the large-diameter portion 4a at the top thereof is covered with the covering 6. Next, the constricting member 9, which is stretched open larger than the outer diameter of the large-diameter portion 4a, is placed on the large diameter portion 4a from above the covering 6. Then, by releasing the constricting member 9 at the position of the circumferential groove 8 formed in the large-diameter portion 4a, the covering 6 is pinched between the constricting member 9 and the large-diameter portion 4a by the elastic restoring force of the constricting member 9, and the constricting member 9 engages with the circumferential groove 8, the covering 6 being disposed therebetween. Accordingly, the constricting member 9 is prevented from moving in the axial direction, and the covering 6 is securely held on the outer circumferential surface of the objective lens 4 by the frictional force between the constricting member 9 and the circumferential groove 8.

As shown in Fig. 2, by applying tension to the covering 6 when the covering 6 is secured by the constricting member 9, it is possible to bring the covering 6 into close contact with the front surface of a front lens 4b disposed at the end surface 5 of the objective lens 4. Because the covering 6 has a thickness of several micrometers to several hundred micrometers, bringing it into close contact with the front surface 4b of the objective lens 4 forms a flat surface that follows the shape of the front surface. Therefore, light passing therethrough does not experience refraction or the like.

In this state, the tip of the objective lens 4 is brought into contact with a specimen (not shown in the drawing) or is inserted inside a specimen, and observation is carried out. By doing so, because the end surface 5 and the outer circumferential surface of the objective lens 4 are covered by the covering 6, as described above, the specimen can be prevented from coming into direct contact with the front surface of the objective lens 4. Therefore, it is possible to prevent problems such as the front surface of the objective lens 4 becoming contaminated by contact with the specimen, or bacteria and the like contained in the specimen becoming adhered to the objective lens 4. In addition, because the covering 6 has no effect on the light passing through the objective lens 4, as described above, it is possible to acquire a clear image.

After observation, it is possible to remove the used covering 6 to which the specimen has adhered simply by removing the constricting member 9 and taking off the covering 6 from the outer surface of the objective lens 4. Cleaning, disinfecting, sterilizing and so forth of the objective lens 4 can thus be eliminated. Therefore, it is possible to omit the procedures for removing the objective lens 4 from the microscope main body 3 and attaching it again, which allows the preparation procedure for observation to be simplified. In addition, it is possible to prevent an external force being applied to the outer surface of the objective lens 4 during these procedures, which can prevent parts that are susceptible to external force, especially the small-diameter tip 2, from being damaged.

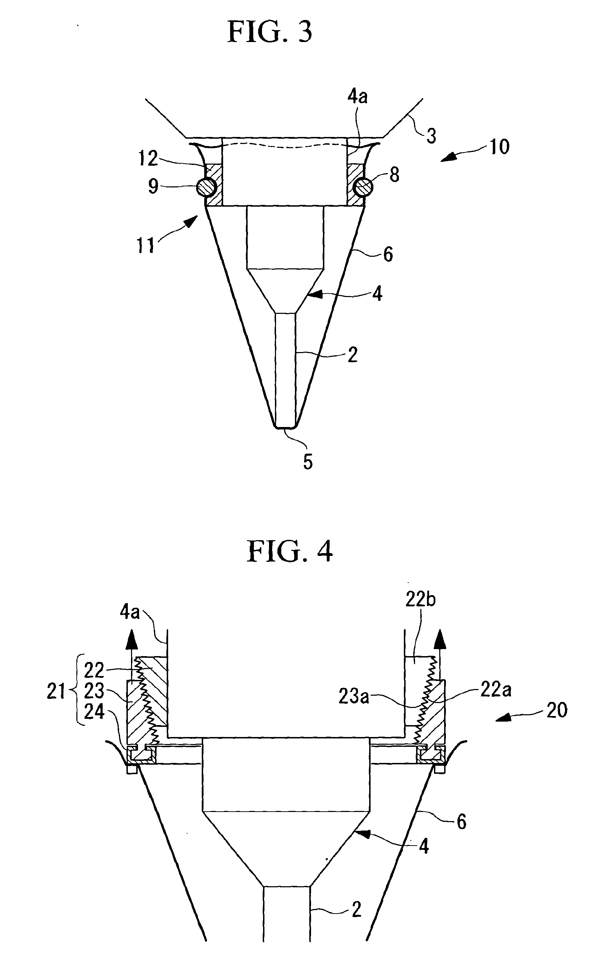

Next, an objective protector 10 according to a second embodiment of the present invention will be described below with reference to Fig. 3.

In the description of this embodiment, parts having the same configuration as those of the objective protector 1 according to the first embodiment described above are assigned the same reference numerals, and a description thereof is omitted.

The difference between the objective protector 10 according to this embodiment and the objective protector 1 according to the first embodiment is an attaching mechanism 11.

As shown in Fig. 3, the attaching mechanism 11 in this embodiment includes a ring-shaped member 12 disposed so as to fit with a large-diameter portion 4a of an objective lens 4. The ring-shaped member 12 may be attached to the objective lens 4 with a bonding agent, or it may be removably secured to the objective lens 4 by means of a push screw (not shown).

A circumferential groove 8 is formed in the outer circumferential surface of the ring-shaped member 12 around the entire circumference thereof, and a constricting member 9 is engaged therewith to pinch the covering 6 therebetween.

This embodiment affords similar advantages to those of the first embodiment. Namely, it is possible to prevent bacteria or contamination from adhering to the outer circumferential surface of the objective lens 4. In addition, cleaning, disinfecting, and sterilizing procedures, as well as various procedures associated therewith, can be omitted, and the objective lens 4 can be protected. Furthermore, because the circumferential groove 8 is formed in the ring-shaped member 12 which is fitted to the outer circumferential surface of the objective lens 4, it is not necessary to form the circumferential groove 8 in the objective lens 4 itself. Therefore, it is possible to attach the covering 6 even to an objective lens 4 which has no circumferential groove 8. By providing the circumferential groove 8 in the ring-shaped member 12, this embodiment can be employed even in cases where there is no space for providing the circumferential groove 8 in the thickness direction of the lens barrel of the objective lens 4.

Next, an objective protector 20 according to a third embodiment of the present invention will be described below with reference to Fig. 4.

The objective protector 20 according to this embodiment differs from the objective protector 10 according to the second embodiment in the structure of a ring-shaped member 21 attached to an objective lens 4.

In this embodiment, the ring-shaped member 21 includes a first ring-shaped member 22 which is secured to the outer surface of a large-diameter portion 4a of the objective lens 4; a second ring-shaped member 23 having a tapered female-threaded portion 23a which is engaged with a tapered male-threaded portion 22a provided on the outer surface of the first ring-shaped member 22; and a third ring-shaped member 24 which is attached to the bottom surface of the second-ring-shaped member 23 in such a manner that it can be moved relative thereto in the circumferential direction.

The first ring-shaped member 22 includes a slotted portion 22b which is axially cut at one place in the circumferential direction. Therefore, by increasing and decreasing the width of the slotted portion 22b, it is possible to increase and decrease the inner diameter.

An outer circumferential portion of the covering 6 is secured in the third ring-shaped member 24.

The operation of the objective protector 23 according to this embodiment, having such a configuration, will be described below.

To attach the objective protector 20 according to this embodiment to the objective lens 4, the slotted portion 22b in the first ring-shaped member 22 is opened to increase the inner diameter and is fitted to the large-diameter portion 4a of the objective lens 4. Then, once the axial position of the first ring-shaped member 22 has been aligned at an appropriate position, the tapered female-threaded portion 23a of the second ring-shaped member 23 is engaged with the tapered male-threaded portion 22a on the outer surface of the first ring-shaped member 22.

When the second ring-shaped member 23 is rotated about the axis of the objective lens 4, the second ring-shaped member 23 moves in the axial direction relative to the first ring-shaped member 22 due to the engagement of the tapered male-threaded portion 22a and the tapered female-threaded portion 23a. Accordingly, the first ring-shaped member 22 is constricted inwards in the radial direction as the engagement proceeds and eventually becomes tightly secured to the objective lens 4 by substantially increasing the friction between itself and the outer circumferential surface of the large-diameter portion 4a. On the other hand, the second ring-shaped member 23 gradually moves towards the microscope main body 3 relative to the first ring-shaped member 22.

Because the third ring-shaped member 24 is attached so as to be capable of rotating relative to the second ring-shaped member 23, it does not rotate even though the second-ring-shaped member 23 rotates. When the second ring-shaped member 23 moves in the axial direction relative to the first ring-shaped member 22, the third ring-shaped member 24 also moves axially together with the second ring-shaped member 23. As a result, by pulling back the covering 6, which is secured to the third ring-shaped member 24, in the axial direction, tension is applied to the covering 6, and the covering 6 is brought into close contact with the front surface of the front lens 4b disposed at the end surface 5 in the objective lens 4.

Therefore, according to this embodiment, the covering 6 can be attached to the objective lens 4 simply by rotating the second ring-shaped member 23 about the axis of the objective lens 4, and the covering 6 can be brought into close contact with the front surface of the front lens 4b.

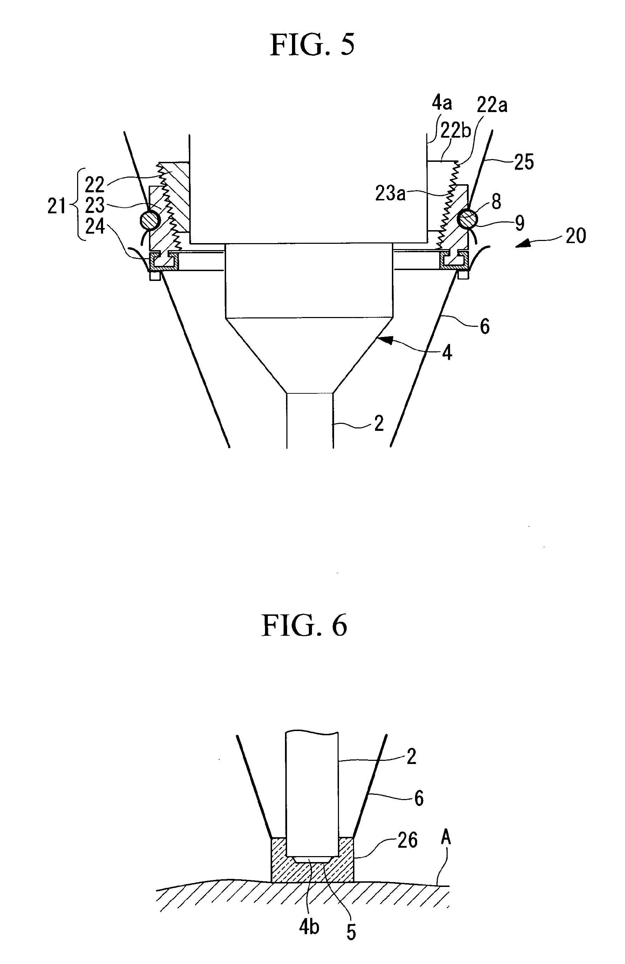

As shown in Fig. 5, by forming a circumferential groove 8 in the outer surface of the second ring-shaped member 23, it is possible to secure a second covering 25 (drape), for covering parts on the microscope main body 3 side, in the circumferential groove 8 in the second ring-shaped member 23 using a constricting member 9. In such a case, it is possible to protect the entire microscope, including not only the objective lens 4 but also the microscope main body 3, from damage or to prevent bacteria adhering thereto.

Although a membrane member is employed as the covering 6 in the embodiments described above, as shown in Fig. 6, it is also possible to dispose a cover member 26, made from optically transparent glass or plastic, at a position where it touches the end surface 5 of the objective lens 4. By doing so, it is possible to easily set the distance between the specimen A and the end surface 5 of the objective lens 4 to match the working distance of the objective lens 4. In this case, it is possible to secure the cover member 26 to the objective lens 4 with wire or the like, without employing a membrane member.

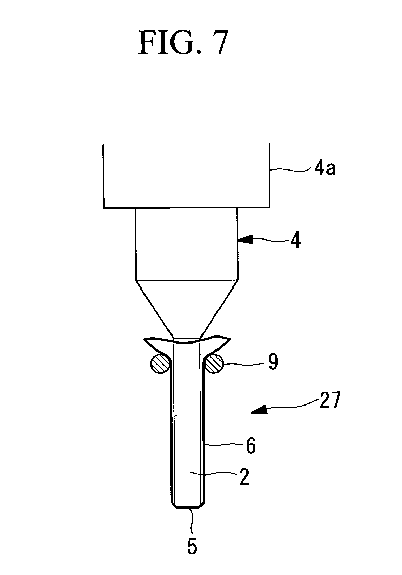

The objective protectors 1, 10, and 20 are secured to the large-diameter portion 4a of the objective lens 4 in the embodiments described above; instead of this, however, an objective protector may be secured to a small-diameter tip 2 of the objective lens 4, as shown in Fig. 7. With this configuration, the covering 6 can be disposed around the outer surface of the small-diameter tip 2 without spreading out into the periphery of the small-diameter tip 2. Therefore, it is possible to ensure that the covering 6 does not act as an obstruction when inserting the objective lens 4 into a small incision made in the specimen.

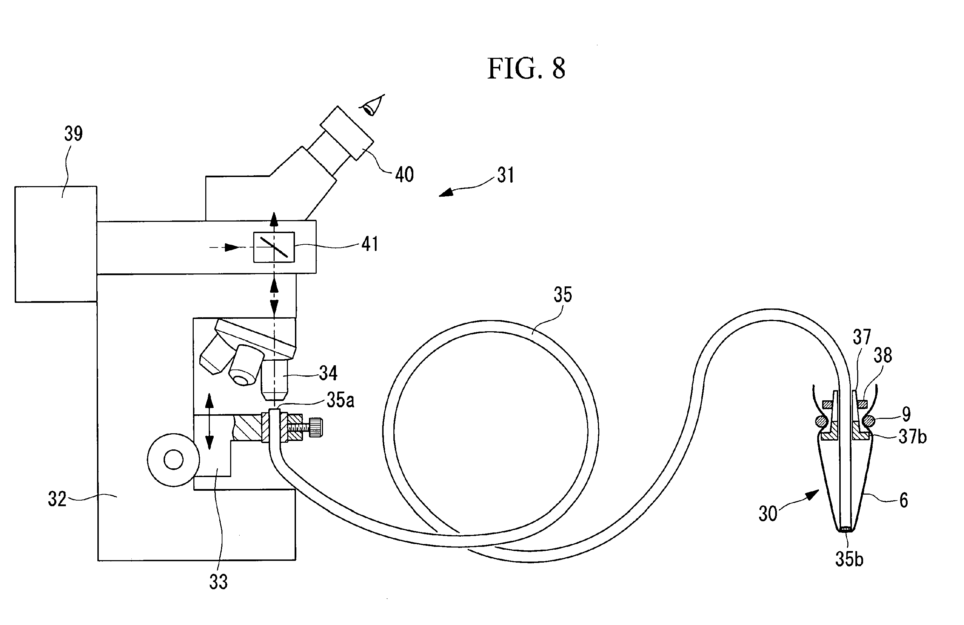

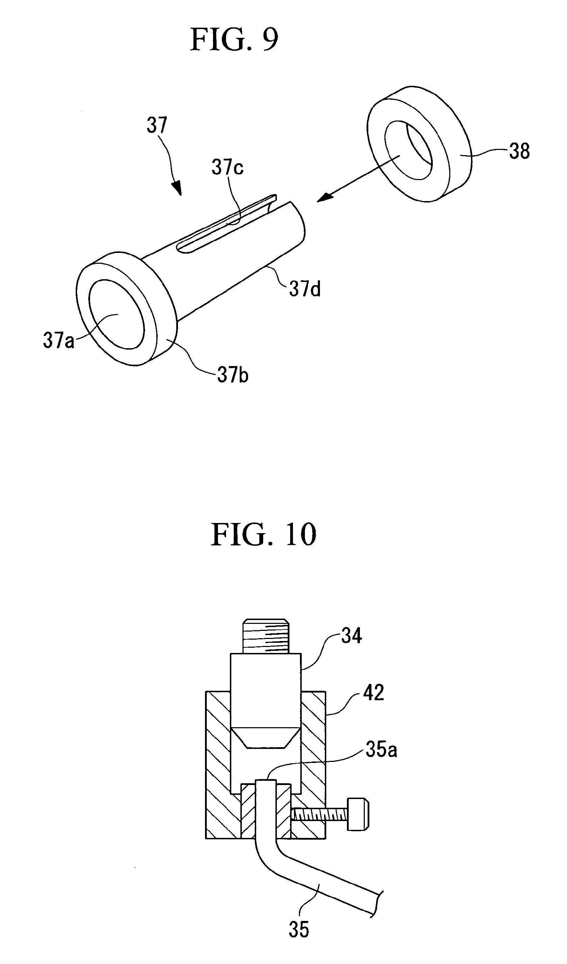

Next, an objective protector 30 according to a fourth embodiment of the present invention will be described below with reference to Figs. 8 and 9.

As shown in Fig. 8, the objective protector 30 according to this embodiment protects at least an end surface 35b of a fiber bundle 35, whose tip is disposed in the vicinity of a focal point of an objective lens 34, using a bracket 33 which is secured to a base 32 of a microscope 31. The objective protector 30 includes a covering 6 formed of a membrane member, and an attaching mechanism 36 which secures the covering 6 to the end of the fiber bundle 35.

As shown in Fig. 9, the attaching mechanism 36 is provided with, for example, a through-hole 37 through which the fiber bundle 35 passes and includes a sleeve 37 whose outer surface is tapered and an engaging ring 38 which is fitted to the outer surface of the sleeve 37. A collar 37b is formed at one end of the sleeve 37, and a slotted portion 37c which extends in the axial direction from the central position in the longitudinal direction is formed at the other end thereof. When the engaging ring 38 is fitted to the sleeve 37, the engaging ring 38 presses the tapered outer surface 37d inwards in the radial direction, whereupon the slotted portion 37c is reduced in size, and by doing so, the sleeve 37 is secured such that it squeezes the outer surface of the fiber bundle 35.

Because a circumferential groove is formed at the outer circumferential surface of the attaching mechanism 36 by the collar 37b of the sleeve 37 and the engaging ring 38, when the sleeve 37 is secured to the outer surface of the fiber bundle 35 by fitting the engaging ring 38 to the sleeve 37, it is possible to attach the covering 6 to the end of the fiber bundle 35 by positioning an O-ring constricting member 9 so as to pinch the covering 6.

Reference numeral 39 in Fig. 8 is a light source such as a halogen lamp, reference numeral 40 is an eyepiece lens, and reference numeral 41 is a dichroic mirror.

With this embodiment, the end of the fiber bundle 35, which is placed in contact with or inserted into the sample, is protected by the objective protector 30. Therefore, procedures for cleaning, disinfecting, or sterilizing the fiber bundle 35 after observation are not necessary, and it is thus possible to simplify the preparation procedure for observation.

Although an example has been given in which the fiber bundle 35 is secured to the base 32 of the microscope 31 with the bracket 33, instead of this, it is also possible to directly secure the fiber bundle 35 to the objective lens 34 using a bracket 42, as shown in Fig. 10.

## Claims

1. An objective protector comprising:
an optically transparent covering configured to cover at least an end surface of an objective lens; and
an attaching mechanism configured to attach the covering to the objective lens.

2. An objective protector according to Claim 1, wherein the covering includes a cover member that is brought into close contact with the end surface of the objective lens.

3. An objective protector according to Claim 1, wherein the covering includes a membrane member that is brought into close contact with the end surface of the objective lens and that is made from a plastic material selected from the group consisting of polyethylene, polyvinylidene chloride, polystyrene, polypropylene, and polycarbonate.

4. An objective protector according to Claim 3, wherein
a circumferential groove is formed in an outer circumferential surface of the objective lens, and
the attaching mechanism includes a constricting member made from a ring-shaped elastic body configured to engage with the circumferential groove to pinch the membrane member between the constricting member and the outer circumferential surface of the objective lens.

5. An objective protector according to Claim 3, wherein the attaching mechanism includes
a ring-shaped member secured to the outer circumferential surface of the objective lens; and
a ring-shaped constricting member configured to engage with the ring-shaped member to pinch the membrane member between the constricting member and the ring-shaped member.

6. An objective protector according to one of Claims 3 to 5, further comprising:
a tension-applying mechanism configured to apply tension to the membrane member so that the membrane member is brought into close contact with the end surface of the objective lens.

7. An objective protector comprising:
an optically transparent covering configured to cover at least an end surface of a fiber bundle, which is disposed at the tip of an objective lens; and
an attaching mechanism configured to attach the covering to the fiber bundle.

8. A microscope observation method comprising:
covering an objective lens provided in a microscope with a membrane member made of an optically transparent plastic material selected from the group consisting of polyethylene, polyvinylidene chloride, polystyrene, polypropylene, and polycarbonate; and
carrying out observation with the membrane member being in close contact with an end surface of the objective lens.

9. An objective lens system comprising:
an objective lens; and
an objective protector attached to the objective lens, wherein the objective protector includes
an optically transparent covering configured to cover at least an end surface of the objective lens, and
an attaching mechanism configured to attach the covering to the objective lens.

10. An objective lens system according to Claim 9, wherein
the covering includes a cover member which is brought into close contact with the end surface of the objective lens.

11. An objective lens system according to Claim 9, wherein
the covering includes a membrane member which is brought into close contact with the end surface of the objective lens and which is made from a plastic material selected from the group consisting of polyethylene, polyvinylidene chloride, polystyrene, polypropylene, and polycarbonate.

12. An objective lens system comprising:
an objective lens;
a fiber bundle disposed at a tip of the objective lens; and
an objective protector attached to the fiber bundle, wherein the objective protector includes
an optically transparent covering configured to cover at least an end surface of the fiber bundle, and
an attaching mechanism configured to attach the covering to the fiber bundle.

13. A microscope system comprising:
a microscope;
an objective lens mounted to the microscope; and
an objective protector attached to the objective lens, wherein the objective protector includes
an optically transparent covering configured to cover at least an end surface of the objective lens, and
an attaching mechanism configured to attach the covering to the objective lens.

14. A microscope system according to Claim 13, wherein
the covering includes a cover member which is brought into close contact with the end surface of the objective lens.

15. A microscope system according to Claim 13, wherein
the covering includes a membrane member which is brought into close contact with the end surface of the objective lens and which is made from a plastic material selected from the group consisting of polyethylene, polyvinylidene chloride, polystyrene, polypropylene, and polycarbonate.

16. A microscope system comprising:
a microscope;
an objective lens mounted to the microscope;
a fiber bundle disposed at a tip of the objective lens; and
an objective protector attached to the fiber bundle, wherein the objective protector includes
an optically transparent covering configured to cover at least an end surface of the fiber bundle, and
an attaching mechanism configured to attach the covering to the fiber bundle.
